# EUROPÄISCHE PATENTANMELDUNG

(11) **EP 3 121 285 A1**
(43) Veröffentlichungstag der Anmeldung: **25.01.2017**
(21) Anmeldenummer: 16188442.4
(22) Anmeldetag: 30.04.2010
(51) Int. Cl.: C12P 7/18, C12P 7/14, C12P 19/14, C12P 5/02

(54) **VERFAHREN ZUR HERSTELLUNG VON XYLITOL AUS LIGNOCELLULOSEHALTIGER BIOMASSE, UMFASSEND EINEN SCHRITT DER ALKOHOLISCH-ALKALINEN DELIGNIFIKATION**

(30) Priorität: 06.08.2009 AT 12522009; 23.09.2009 AT 14962009; 23.12.2009 AT 20302009
(62) Teilanmeldung aus: 10717481.5
(71) Anmelder: Annikki GmbH, 8020 Graz (AT)
(72) Erfinder: Fackler, Karin, 1140 Wien (AT); Messner, Kurt, 1170 Wien (AT); Sakdaronnarong, Chularat, 11140 Nonthaburi (TH); Ertl, Ortwin, 8076 Vasoldsberg (AT)
(74) Vertreter: Nemec, Harald

(57) **Zusammenfassung**

Die Erfindung betrifft ein Verfahren zur Herstellung von Xylitol, gekennzeichnet durch die Kombination der Maßnahmen, dass
- lignocellulosisches Material mit einer wässrigen Lösung, welche einen Alkohol, insbesondere einen C₁₋₄-Alkohol oder ein Phenol, enthält und einen pH-Wert zwischen 11,0 und 14,0 aufweist, behandelt wird, um Lignocellulose zu spalten, und Spaltprodukte aus dem Material abzutrennen, wobei ein mit Cellulose und Hemicellulose angereichertes Material erhalten wird,
- das erhaltene, mit Cellulose und Hemicellulose angereicherte Material mit Xylanase behandelt wird um Xylose zu gewinnen, und
- die Xylose enzymatisch zu Xylitol umgewandelt wird.

## Beschreibung

Die vorliegende Erfindung betrifft ein Verfahren zur Herstellung von Kohlenhydratspaltprodukten, insbesondere Zuckern , wie Pentosen und Hexosen, aus lignocellulosischem Material. Die Erfindung betrifft ferner ein Verfahren zur Gewinnung von Alkohol aus Zuckern. Für die Zwecke der vorliegenden Beschreibung und Patentansprüche soll der Begriff "Zucker" auch "Zucker-Oligomere" umfassen.

Im Zusammenhang mit der Verknappung von Rohöl und der Diskussion um Getreide als Energielieferant gewinnt der nachwachsende Rohstoff Lignocellulose (Stroh, Holz, Papierabfälle etc.) als Ausgangsmaterial für Treibstoffe oder chemische Produkte sehr an Bedeutung. Die Konversion der Lignocellulose kann nach zwei grundlegend verschiedenen Wegen erfolgen: 1) der "Thermochemical Platform", bei der die Lignocellulose zuerst vergast und die Synthesegase zu gewünschten Produkten synthetisiert werden, und 2) die "Sugar Platform", bei der das Hauptinteresse in der Nutzung der in den Polymeren Cellulose und Hemicellulosen gebundenen Zucker besteht, während Lignin noch vorwiegend energetisch genutzt wird. Die vorliegende Erfindung ist dem zweiten Weg zuzuordnen.

Im Gegensatz zur Stärke liegen die Zucker der Lignocellulose in eng vernetzten, polymeren, kristallinen Strukturen der Cellulose und Hemicellulosen vor, die zusätzlich von einem Ligninmantel umhüllt sind, wodurch sich ein äußerst dichter Komplex ergibt. Der naheliegendste Weg, um aus Lignocellulose Zucker zu gewinnen, wäre der direkte Einsatz von Cellulasen und Hemicellulasen. Dies wird jedoch am Rohstoff Stroh oder Holz durch die Dichte des oben erwähnten Komplexes erschwert. Durch ihr hohes Molekulargewicht sind Enzyme nicht imstande durch die engen Poren in die Lignocellulose einzudringen. Dies bedeutet, dass ein erster Schritt gesetzt werden muss, der die Porosität der Lignocellulose erhöht und dadurch die weitere enzymatische Verzuckerung ermöglicht.

Dieser erste Schritt wird als "Pretreatment" (Vorbehandlung, Aufschluss) bezeichnet. Er ist durchwegs sehr aufwändig, sodass z.B. bei der Herstellung von "second generation biofuels" bis zu 1/3 der Produktionskosten dafür aufgewendet werden müssen, was die Rentabilität negativ beeinflusst. Die angewandten Verfahren zielen entweder darauf ab, primär die Hemicellulosen zu verflüssigen (z.B. steam explosion-, dilute acid-pretreatment) oder die Erhöhung der Porosität durch Verflüssigung von Lignin (z.B. lime-, ammonia-pretreatment) zu erreichen.

Das aufgeschlossene Lignocellulose-Substrat kann zur Gewinnung von Zuckern bzw. ihrer Oligomere enzymatisch weiterbehandelt werden, wobei die Art der Vorbehandlung starken Einfluss auf die Enzymaktivität und die Ausbeute haben kann. Bei hohen Reaktionstemperaturen entstehen vielfach toxische Abbauprodukte (z.B. Furfural), welche im Falle einer unmittelbar angeschlossenen Ethanol-Gärung, die Hefen hemmen können; siehe z. B. Chandra et al., Advances in Biochemical Engineering/Biotechnology, 108:67, 2007; Mansfield et al., Biotechnol.Prog. 15:804, 1999.

Diese Verfahren haben den gravierenden Nachteil, dass sie energieaufwändig sind und vorwiegend bei Temperaturen knapp unter 200°C ablaufen.

Eine technologische Verbesserung in diesem Bereich, z.B. durch die Entwicklung von Niedertemperaturverfahren, (d.h. bei einer Temperatur von unter 100°C), würde einen entscheidenden Fortschritt bei jeglicher stofflicher Nutzung des Rohstoffes Lignocellulose bedeuten. Dies ist die Aufgabe der vorliegenden Erfindung.

Aus der EP 1 025 305 B1 ist ein chemisches Verfahren zur Lignin-Depolymerisation (Cu-System) bekannt. Es beruht auf der katalytischen Wirkung von komplexiertem Kupfer in Verbindung mit Wasserstoffperoxid oder organischen Hydroperoxiden und ist imstande, Lignin bei Temperaturen unter 100°C oxidativ zu spalten. Die dabei eingesetzten Komplexbildner sind Pyridin-Derivate. An synthetischen Ligninmodellen konnte nachgewiesen werden, dass bei Verwendung von H₂O₂ als Oxidationsmittel eine Spaltung von Etherbindungen des Ligninmoleküls erfolgt, wodurch das Ligninpolymer zu oligomeren Untereinheiten zerfällt. Bei Einsatz des Cu-Systems mit einem Überschuss an organischen Hydroperoxiden ist es möglich, Holz zu delignifizieren. Das auf H₂O₂ basierte System erscheint technisch besser umsetzbar zu sein, wurde als Bleichzusatz bei der Peroxidbleiche von Kraft-Zellstoff getestet und führte zu einer verbesserten Delignifizierungsrate und höherem Weißegrad.

Ferner ist aus "Oxidation of wood and its components in water-organic media", Chupka et al., Proceedings: Seventh International symposium on wood and pulping chemistry, Vol. 3, 373-382, Beijing P.R. China, 25.-28.Mai 1993, bekannt, dass die Effizienz einer alkalischen Katalyse der Oxidation von Holz und Lignin beträchtlich zunimmt, wenn dem wässrigen Reaktionsmedium ein organisches Lösungsmittel, z.B. DMSO, Aceton, Ethanol, zugegeben wird. Ferner geben die Autoren an, dass bei pH-Werten über 11 ein scharfer Anstieg der Oxidation des Holzes und des Lignins stattfindet.

Aus der WO 01/059204 ist ein Verfahren zur Herstellung von Zellstoff bekannt, bei dem das Ausgangsmaterial einer Vorbehandlung unterzogen wird, wobei das Material mit einer Pufferlösung und einem Delignifizierungskatalysator (Übergangsmetall) behandelt wird. Die Delignifizierung wird in Gegenwart von Sauerstoff, Wasserstoffperoxid oder Ozon durchgeführt.

Demgegenüber ist das erfindungsgemäße Verfahren zur Herstellung von Xylitol gekennzeichnet durch die Kombination der Maßnahmen, dass
- lignocellulosisches Material mit einer wässrigen Lösung, welche einen Alkohol, insbesondere einen C₁₋₄-Alkohol oder ein Phenol, enthält und einen pH-Wert zwischen 11,0 und 14,0 aufweist, behandelt wird, um Lignocellulose zu spalten, und Spaltprodukte aus dem Material abzutrennen, wobei ein mit Cellulose und Hemicellulose angereichertes Material erhalten wird,
- das erhaltene, mit Cellulose und Hemicellulose angereicherte Material mit Xylanase behandelt wird um Xylose zu gewinnen, und
- die Xylose enzymatisch zu Xylitol umgewandelt wird.

Bevorzugte Ausführungsformen sind in den Unteransprüchen angeführt.

Als Alkohol eignen sich aliphatische oder cycloaliphatische, ein- oder mehrwertige Alkohole oder Phenole, z. B. C₁₋₆-Alkohole, insbesondere ein C₁₋₄-Alkohol, wie Methanol, Ethanol,

Propanol und Butanol, inklusive deren Isomere; Glycole (Ethandiole, Propan-, Butan-, Pentan-, Hexandiole), Glycerin, Propenol, Butenol, Cyclopentanol, Cyclohexanol, Benzylalkohol; oder Phenole, wie Phenole, Cresole, Catechole, Naphthole, aber auch Aminoalkohole, wie Ethanolamin, Methanolamin und Hexanolamin. Bevorzugt ist ein C₁₋₄-Alkohol. Für die Zwecke der vorliegenden Patentanmeldung sind Phenole in den Gattungsbegriff "Alkohol" mit eingeschlossen.

Die alkoholische Lösung des Lignin-Extraktes bietet außerdem vorteilhafte Optionen in der weiteren Aufarbeitung der Lignin-, bzw. Xylan-Spaltprodukte.

Alkohol liegt in einer wässrigen Lösung im erfindungsgemäßen Verfahren bevorzugt in einem Ausmaß von 10 bis 70 Vol%, z. B. 20 bis 50 Vol%, bevorzugt von 30 bis 40 Vol% vor.

Im erfindungsgemäßen Verfahren liegt das lignocellulosische Material in der wässrigen Lösung vorzugsweise in einer Stoffdichte von 3-40 Gew%, wie 5-40 Gew%, insbesondere 5-20% Gew% vor.

Bevorzugt wird die Lignocellulose bei einer Temperatur von unter 100°C, wie unter 80°C, z. B. unter 60°C gespalten.

Der pH Wert kann mit einer Base, bevorzugt einer anorganischen Base, beispielweise Natronlauge eingestellt werden.

Die vorliegende Erfindung beruht auf der Erkenntnis, dass ein mit einer wässrigen, basischen Lösung, welche einen Alkohol, insbesondere einen C₁₋₄-Alkohol oder ein Phenol, enthält und einen pH-Wert zwischen 11,0 und 14,0 aufweist, behandeltes lignocellulosisches Material enzymatisch in höherer Ausbeute zu Kohlenhydratspaltprodukten, wie Zuckern, verarbeitet werden kann, als ein auf eine sonstige Art delignifiziertes Material, insbesondere ohne den Zusatz von Alkohol.

Als Kohlenhydratspaltprodukte werden hauptsächlich Pentosen und Hexosen gebildet. Bevorzugte Zucker schließen Xylose und Glucose ein.

Eine bevorzugte Ausführungsform des erfindungsgemäßen Verfahrens ist dadurch gekennzeichnet, dass das mit Cellulose und Hemicellulose angereicherte Material mit einer Xylanase und einer Cellulase behandelt wird, um die Zucker zu gewinnen.

Als lignocellulosisches Material wird vorzugsweise Stroh, Energiegräser, wie z. B. Switchgrass, Elefantengras oder Abaca, Sisal, Bagasse, oder untypische Lignocellulosesubstrate, wie Spelzen, z.B. Reisspelzen, bevorzugt Stroh, Energiegräser, Bagasse oder Spelzen, besonders bevorzugt Stroh oder Bagasse, z. B. Stroh, eingesetzt. Stroh hat eine stark hydrophobe Oberfläche, sodass die Benetzung mit wässrigen Lösungen ein Problem darstellt. Es hat sich gezeigt, dass es durch die Verwendung von Alkohol möglich ist, selbst ohne Druck die Reaktionslösung in die Poren des Substrates einzubringen und die vorhandene Luft durch Reaktionslösung zu ersetzen. Ferner hat sich gezeigt, dass Alkohol die Extraktion der Spaltprodukte aus Stroh beschleunigt und dazu beiträgt, die Ligninspaltprodukte in Lösung zu halten. Weiters hat sich gezeigt, dass im Gegensatz dazu Alkohol die Löslichkeit der Hemicellulose und deren Spaltprodukte herabsetzt und somit die Hemicellulose im Substrat gehalten wird.

Durch Abpressen der flüssigen Phase vom Substrat nach dem Aufschlussverfahren wird die Substratkonzentration erhöht, sodass geringere Enzymmengen zur enzymatischen Hydrolyse, bzw. bei anderen enzymatischen Weiterverarbeitungen erforderlich sind.

Bei der Alkoholproduktion sind die Enzymkosten ein entscheidender Kostenfaktor. Alkohol führt dazu, dass die Löslichkeit der im alkalischen Bereich während der Reaktion zusätzlich zum Lignin eventuell freigesetzten Hemicellulosen und deren Spaltprodukte stark herabgesetzt wird und diese an das Substrat gebunden bleiben. Die Vorteile für den Prozess sind hohe Selektivität des Ligninabbaus, für den Fall einer Abtrennung der Extraktionslösung vom Feststoff eine sehr geringe Konzentration an Hemicellulose und deren Spaltprodukten in der Extraktionslösung, denn die Hemicellulose bleibt im Feststoffanteil und dadurch für die enzymatische Hydrolyse und Zuckergewinnung erhalten. Die alkoholische Lösung des Lignin-Extraktes bietet ferner verbesserte Möglichkeiten in der weiteren Aufarbeitung des Lignins und der Herstellung von Produkten aus Lignin.

Es hat sich weiters gezeigt, dass durch die Verwendung von Alkohol, insbesondere eines C₁₋₄-Alkohols oder eines Phenols, beim alkalischen Aufschluss unter 100°C der Abbau der Hemicellulosen weitgehend verhindert wird, sodass annähernd die gesamte Hemicellulose zur weiteren enzymatischen Spaltung und Umwandlung der Xylose zu höherwertigen Produkten zur Verfügung steht und nicht, wie bei anderen Verfahren, teilweise beim Aufschluss mit abgebaut wird und als Lignin / Zucker-Mischung anfällt.

Durch die im Aufschluss durchgeführte Delignifizierung wird die Porosität der Zellwände des lignocellulosischen Materials erhöht, beispielsweise im Falle von Stroh so weit erhöht, dass nahezu das gesamte Xylan für die Xylanase zugänglich wird und annähernd 100% des Xylans hydrolysiert und Xylose gewonnen werden kann. Dies macht das Verfahren gemäß der vorliegenden Erfindung besonders geeignet, in Verbindung mit einer enzymatischen Konversion der Xylose höherwertige Produkte herzustellen. Die enzymatische Konversion kann dabei entweder direkt im Gemisch aus Xyloselösung und Feststoff erfolgen, oder aber mit der vom Feststoff abgetrennten Xyloselösung.

Bei einer weiteren, nach der enzymatischen Hydrolyse des Xylans und der erfindungsgemäßen Umwandlung der Xylose zu Xylitol folgenden Alkoholproduktion aus dem verbleibendem Feststoff, sind die Enzymkosten ein entscheidender Kostenfaktor. Diese resultieren zum Teil auch aus unspezifischen Bindungen von Enzymen an das Lignin, siehe z B. Chandra et al, 2007, ibidem. Die teilweise Entfernung des Lignins reduziert diesen Aktivitätsverlust und wirkt sich kostengünstig aus.

Die Vorteile für ein nachfolgendes enzymatisches Verfahren sind beispielsweise, dass aus der hohen Selektivität des Ligninabbaus bei fast vollständiger Erhaltung der Zuckerpolymere eine sehr geringe Konzentration an Hemicellulose und deren Spaltprodukten in der Extraktionslösung resultiert, die Hemicellulose bleibt im Feststoffanteil und dadurch für die enzymatische Hydrolyse und Zuckergewinnung sowie deren weiterer Umwandlung erhalten.

Daraus ergibt sich erfindungsgemäß eine maximale Stoffnutzungsrate und, beispielsweise in Verbindung mit dem Einsatz von Xylosedehydrogenasen, hohe Rentabilität des beschriebenen Prozesses.

Die Durchführung eines Xylose-Umwandlungsprozesses zu Xylitol kann nach enzymatischer Freisetzung der Xylose direkt im Feststoff/Flüssigkeitsgemisch, das gemäß dem vorliegenden erfindungsgemäßen Verfahren erhalten wird, durchgeführt werden, was weiterhin die Rentabilität des Gesamtprozesses erhöht.

Im Falle der Umwandlung zu Xylitol kann der, nach Abpressen des Feststoffes im Substrat verbleibende, Restalkohol aus dem Aufschlussprozess direkt als Substrat für die Alkoholdehydrogenase zur Regenerierung von NAD⁺ zu NADH genutzt werden. Wenn der Prozess so ausgelegt wird, dass dazu der im Reaktionsgemisch verbleibende Restalkohol aus dem Aufschluss (teilweise) verbraucht wird, wird eine Alkoholentfernung aus der Produktlösung (teilweise) überflüssig und die Effizienz des Gesamtprozesses dadurch weiter gesteigert.

Im Falle der Umwandlung der Ligninspaltprodukte wirkt der Alkohol als Radikal-Scavenger und Lösungsmittel für Spaltprodukte aus einer enzymatischen, biomimetischen oder chemischen Depolymerisation der höhermolekularen Lignin-Spaltprodukte zu niedermolekularen.

Der geringe Anteil von Hemicelluose und deren Spaltprodukten im Extrakt und die erhöhte Löslichkeit des Lignins, erhöhen die Durchsatzraten bei einer Abtrennung des Feststoffes von den Umwandlungsprodukten, sowie deren Aufarbeitung durch Filtration.

Das erfindungsgemäße Verfahren erlaubt beispielsweise die Auftrennung der drei Hauptkomponenten des Strohs, nämlich der Glucose, der Xylose sowie des Lignins in sehr störstoffarmen Stoffströmen und deren weitere Umwandlung zu höherwertigen Produkten, wie Xylitol, und erfüllt somit die Forderungen eines idealen Bioraffinerie-Verfahrens.

Ein weiterer Vorteil des erfindungsgemäßen Verfahrens im Vergleich zu anderen Aufschlussverfahren, die vorwiegend im Temperaturbereich zwischen 150°C und 200°C ablaufen, ist seine Reaktionstemperatur unter 100°C. Der niedrige Energieaufwand erlaubt es, das beim Aufschluss gewonnene Lignin nicht als Energiequelle für das Aufschlussverfahren, sondern als Wertstoff zu nutzen.

Nach der Behandlung mit der wässrigen, einen Alkohol, insbesondere einen C₁₋₄-Alkohol oder ein Phenol, und H₂O₂ enthaltenden Lösung, wird, gemäß dem Verfahren der vorliegenden Erfindung, die Lignin enthaltende Lösung abgetrennt und der aufgeschlossene Feststoff bevorzugt mit einer Xylanase, z. B. 6-72 Stunden bei 30-90°C behandelt und die Flüssigphase vom Feststoff abgetrennt, worauf die Flüssigphase bevorzugt zu Folgeprodukten, z. B. Xylitol weiter umgesetzt wird.

Der nach Abtrennung der Flüssigphase verbleibende Feststoff wird bevorzugt mit Cellulase behandelt, wobei durch weitere Fermentation der Feststoff/Glucoselösung Ethanol, Butanol oder andere Fermentationsprodukte erhalten werden können; oder der verbleibende Feststoff wird einer thermischen oder thermochemischen Stoffumwandlung unterzogen und die entstandenen Produkte, wie Treibstoffkomponenten, Treibstoffzusätze und/oder andere Chemieprodukte, wie z. B. Phenole, werden abgetrennt; oder der verbleibende Feststoff wird einer mikrobiellen Stoffumwandlung durch Bakterien, Hefen oder Pilze unterzogen; oder der verbleibende Feststoff wird einem weiteren Delignifizierungsschritt zum Zwecke der Gewinnung eines Cellulose-Fasermaterials unterzogen.

Der verbleibende Feststoff kann in einer Biogasanlage fermentiert und zu Biogas weiterverarbeitet werden.

Eines der wirtschaftlich interessantesten Folgeprodukte der Xylose ist Xylitol.

Die Hauptquellen für die Xylose-Gewinnung sind Kochlaugen aus der Zellstoffindustrie, die eine Fülle von Abbauprodukten, hauptsächlich des Lignins und der Hemicellulose enthalten, sodass Xylose erst durch aufwändige Trennungs- und Reingungsschritte gewonnen werden muss. So beschreibt z. B. H. Harms in "Willkommen in der natürlichen Welt von Lenzing, weltweit führend in der Cellulosefaser Technologie", Herbsttagung der österreichischen Papierindustrie, Frantschach (15. 11. 2007) die Gewinnung von Xylose aus der Dicklauge durch Gelfiltration, eine technisch sehr komplexe Methode, die üblicherweise nicht für Bulkprodukte Anwendung findet. Die solcherart gewonnene Xylose wird dann katalytisch zu Xylitol umgewandelt.

In einem weiteren Aspekt wird die gemäß vorliegender Erfindung gewonnene Xylose fermentationsfrei in Xylitol umgewandelt, durch Umsetzung mit einer Xylosereduktase, z. B. einer Xylose Dehydrogenase, beispielsweise aus *Candida tenuis*, wobei gegebenenfalls eine Xylosereduktase und gegebenenfalls ein Kosubstrat zur Regenerierung des Co-Faktors und gegebenenfalls Alkoholdehydrogenase und gegebenenfalls NAD(P)H zur Xylose Lösung zugesetzt wird; insbesondere, wobei das erhaltene Xylitol durch Filtration von den Ligninspaltprodukten abgetrennt wird.

Mit dem nachstehenden Beispiel 1 und Vergleichsbeispiel 1A wird der Einfluss der Vorbehandlung in Gegenwart von Alkohol auf die Ausbeute an reduzierenden Zuckern nach enzymatischer Hydrolyse dokumentiert.

### Beispiel 1

### Vorbehandlung von Weizenstroh

Weizenstroh wird auf eine Partikelgröße von ca. 2 cm zerkleinert. 5 g zerkleinertes Weizenstroh wird in einem 500 mL Reaktionsgefäß in 200 mL einer Lösung, bestehend aus 49,5% Wasser, 50% Ethanol und 0,5% Wasserstoffperoxid suspendiert. Die Suspension wird auf 50 °C im Wasserbad erhitzt, thermostatisiert und der pH-Wert der Suspension mit wässriger NaOH-Lösung auf einen Ausgangs-pH Wert von 12 eingestellt. Die Mischung wird bei 200 rpm, 60°C, 24 Stunden kontinuierlich magnetisch gerührt. Danach wird der Feststoffanteil abfiltriert und mit 1L destilliertem Wasser gewaschen.

Zur enzymatischen Hydrolyse wurden von jedem Parallelversuch 100 mg vorbehandeltes Substrat mit 9,8 mL 50 mM Na-Acetat Puffer auf pH 4,8 gestellt und mit 200 µL Accellerase 1000 Suspension (www.genencor.com) versetzt. Accellerase ist eine Enzymmischung aus Cellulasen und Hemicellulasen. Die enzymatische Hydrolyse wurde bei 50°C in einem Schüttelwasserbad durchgeführt. Die nach 48 Stunden freigesetzten löslichen Monomere aus Hexosen und Pentosen wurden in Form reduzierender Zucker nach der DNS Methode (Miller et al., Analytical Chemistry 31(3):426, 1959) in 1 mL flüssigem Überstand bestimmt, auf die Menge des eingewogenen, vorbehandelten Substrates bezogen und in Prozent der maximalen theoretischen Ausbeute ausgedrückt.

Die theoretische maximale Ausbeute reduzierender Zucker wurde gesondert bestimmt und beträgt 705 mg +/- 5% pro g unbehandeltes Stroh.

Pro Versuchsansatz wurden jeweils 5 Parallelversuche durchgeführt. Die Ausbeute an reduzierenden Zuckern betrug 99% +/- 4%.

### Vergleichsbeispiel 1A

Das Beispiel 1 wurde wiederholt, jedoch ohne Alkoholzusatz. Die Ausbeute an reduzierenden Zuckern betrug lediglich 64% +/- 3%.

### Beispiel 2

### Vorbehandlung von Weizenstroh

Weizenstroh wird auf eine Partikelgröße von ca. 2 cm zerkleinert. 2,5 g zerkleinertes Weizenstroh wird in einem 500 mL Reaktionsgefäß in 200 mL einer Lösung, bestehend aus 49,5% Wasser und 50% Isopropanol suspendiert. Die Suspension wird auf 50 °C im Wasserbad erhitzt, thermostatisiert und der pH-Wert der Suspension mit wässriger NaOH-Lösung auf einen Ausgangs-pH Wert von 13 (bzw. 14) eingestellt. Die Mischung wird bei 200 rpm, 60°C, 24 Stunden kontinuierlich magnetisch gerührt. Danach wird der Feststoffanteil abfiltriert und mit 1L destilliertem Wasser gewaschen.

Zur enzymatischen Hydrolyse wurden von jedem Parallelversuch 100 mg vorbehandeltes Substrat mit 9,8 mL 50 mM Na-Acetat Puffer auf pH 4,8 gestellt und mit 200 µL Accellerase 1000 Suspension (www.genencor.com) versetzt. Accellerase ist eine Enzymmischung aus Cellulasen und Hemicellulasen. Die enzymatische Hydrolyse wurde bei 50°C in einem Schüttelwasserbad durchgeführt. Die nach 48 Stunden freigesetzten, löslichen Monomere aus Hexosen und Pentosen wurden in Form reduzierender Zucker nach der DNS Methode in 1 mL flüssigem Überstand bestimmt, auf die Menge des eingewogenen vorbehandelten Substrates bezogen und in Prozent der maximalen theoretischen Ausbeute ausgedrückt.

Die theoretische maximale Ausbeute reduzierender Zucker wurde gesondert bestimmt und beträgt 705 mg +/- 5% pro g unbehandeltes Stroh.

Pro Versuchsansatz wurden jeweils 5 Parallelversuche durchgeführt. Die Ausbeuten an reduzierenden Zuckern betrug 97% +/- 4%.

### Beispiel 3

### Enzymatische Xylitol Produktion aus einer Xyloselösung, die aus Stroh nach dem im Beispiel 2 beschriebenen Verfahren hergestellt wurde. Als Kosubstrat wird Isopropanol verwendet.

Die Reaktionslösung enthält 5 mg/mL Xylose.
Xylosereduktase (XR) aus *Candida tenuis* reduziert Xylose zu Xylitol. Diese XR benötigt als Koenzym NADH (Nicotinamidadenindinukleotid reduziert), das bei der Reaktion zum Koenzym NAD⁺ oxidiert wird. Die Regenerierung des oxidierten Kofaktors erfolgt durch parallele Aktivität einer Alkohol-Dehydrogenase (ADH: Enzym-gekoppelte Regenerierung). Als Kosubstrat wird Isopropanol eingesetzt. Isopropanol und NAD⁺ werden durch die ADH zu NADH und Aceton umgesetzt, wie im Reaktionsschema 1 gezeigt:

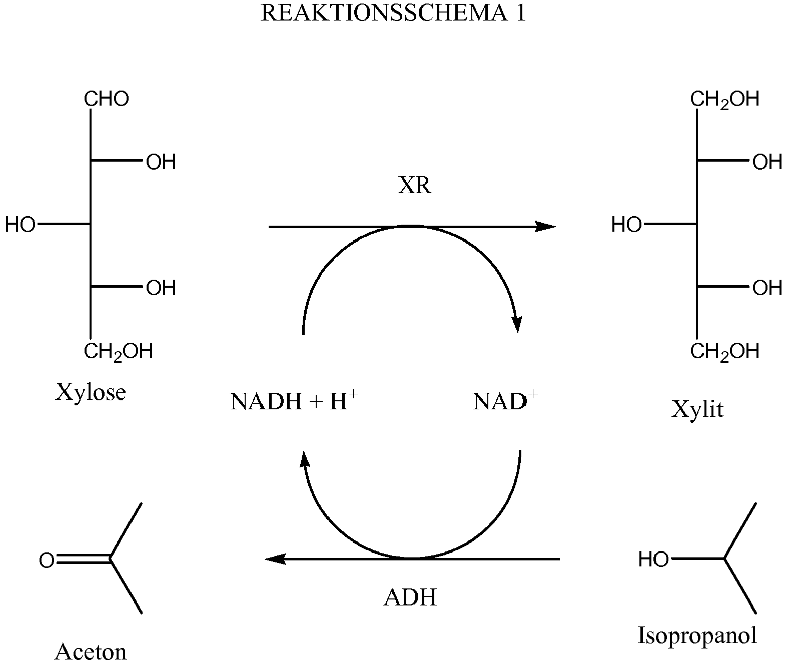

In der Tabelle 1 sind die Reaktionsverhältnisse in den 5 verschiedenen Versuchsreaktionen #049, #050, #051, #052, #053 und #054 dargestellt:

**Tabelle 1**

| **Reaktions Nummer** | **#049** | **#050** | **#052** | **#053** | **#054** |
|---|---|---|---|---|---|
| Substrat batch I [µl] | 250 | 250 | 250 | 500 | 500 |
| XR *C.tenuis* 2 U/mL [µL] | | 50 | 50 | | 50 |
| 20 mM NADH [µL] | | 50 | 50 | | 50 |
| ADH *L.kefir* 5U/mL [µL] | | | 50 | | 50 |
| Isopropanol [µL] | | | 50 | | 50 |
| 50 mM Na-Phosphate Puffer, pH 7.0 [µL] | 750 | 650 | 550 | 500 | 300 |

Gesamtvolumen: 1 mL
Temperatur: 26 ± 2°C
Magnetrührer: 200 rpm
Zeit: 15 Stunden
Zur Deaktivierung der Enzyme wurden alle Proben auf 95°C für 15 Minuten aufgeheizt und als Vorbereitung für die anschließende HPLC-Analyse zentrifugiert.

### Analyse - HPLC:

Säule SUGAR SP0810 + Vorsäule SUGAR SP-G
Detektor: Refraktionsindex-Detektor
Eluent: entionisiertes H₂O
Fluss: 0.75 mL/min
Probenmenge: 10 µL
HPLC Quantifizierung Präzision: ±10%

### Retentionszeit:

Xylose: 13,97 min
Xylitol: 37,73 min
Isopropanol: 16,69 min
Aceton: 16,54 min

### Ergebnisse:

Die Substratkonzentration von Probe #049 wurde mittels HPLC bestimmt und lag bei 0.9 mg/mL.

Die Reaktionsmischung #050 beinhaltet nur Xylosereduktase (0.1 U/mL) und NADH (1 mM). Nach der 15 Stunden dauernden Reaktion waren 0.085 mg Xylose verbraucht. Die Xylitol Konzentration war unter dem Detektionslimit.

Die Reaktion #052 ist vergleichbar mit der Reaktion #050, jedoch mit dem Unterschied, dass hier das Regenerationssystem angewendet wird. Es kommt zum Totalumsatz der eingesetzten Xylose. Verwendete Konzentrationen: XR (0.1 U/mL), NADH (1 mM), ADH (0.25 U/mL) und Isopropanol (5%).

Die Xylosekonzentration der Probe #053 wurde mit 2.121 mg/mL bestimmt, was der erwarteten Xylosekonzentration entspricht.

Die Reaktion #054 ist vergleichbar mit Reaktion #052, beinhaltet jedoch eine um den Faktor 2 erhöhte Xylose-Startkonzentration (50% Substrate in der Reaktion). Die Konzentration des erzeugten Xylitols wurde mit 0.945 mg Xylitol gemessen. Verwendete Konzentrationen: XR (0.1 U/mL), NADH (1 mM), ADH (0.25 U/mL) und Isopropanol (5%).

In der Tabelle 2 sind die Ergebnisse der Reaktionen basierend auf den HPLC-Messdaten zusammengefasst (Xylose verbraucht und Xylitol gewonnen; "u.D.L." bedeutet "unter dem Detektionslimit"):

**Tabelle 2**

| **Reaktionsnummer** | **049** | **050** | **052** | **053** | **054** |
|---|---|---|---|---|---|
| Xylose vor der Reaktion [mg/mL] | 0,9 | 0,815 | 0,8 | 2,121 | 1,945 |
| Xylose nach der Reaktion [mg/mL] | - | 0,815 | u.D.L. | - | 1,013 |
| Xylose verbraucht in der Reaktion [mg/mL] | - | u.D.L. | | - | 0,932 |
| | | | | | |
| Gewinnung von Xylitol [mg/mL] | - | u.D.L. | 0,994 | - | 0,945 |
| Xylitol-Ausbeute relativ zur Xylosekonzentration [%] | - | u.D.L. | 100 | - | 47,9 |

### Beispiel 4

### Enzymatische Xylitol Produktion aus einer Xyloselösung, die aus Stroh nach dem im Beispiel 2 beschriebenen Verfahren hergestellt wurde. Als Kosubstrat wird Ethanol verwendet.

Das Volumen der Substratlösung wurde (vgl. Beispiel 2) mittels eines Rotavapors auf 50% vermindert, um die Xylosekonzentration zu erhöhen (∼10 mg/mL Xylose).

Die Regenerierung des oxidierten Kofaktors erfolgte durch die Aktivität der eingesetzten Xylose-Reduktase (XR) aus *Candida tenius* und die zusätzliche Aktivität einer eingesetzten Aldehyd-Dehydrogenase aus *Saccharomyces cerevisiae* (Sigma-Aldrich: Katalognummer A6338; (EC) Number: 1.2.1.5; CAS Number: 9028-88-0). Dabei handelt es sich sowohl um eine Substrat-gekoppelte, als auch um eine Enzym-gekoppelte Reaktion. Als Kosubstrat wird Ethanol eingesetzt. Ethanol und NAD⁺ werden im ersten Schritt durch die Aktivität der XR zu NADH und Acetaldehyd umgesetzt. Im zweiten Schritt werden Acetaldehyd und NAD⁺ durch die Aktivität der Aldehyd-Dehydrogenase (AldDH) zu Acetat umgesetzt (vgl. dazu Sigma-Aldrich: Katalognummer A6338; bzw. "Characterization and Potential Roles of Cytosolic and Mitochondrial Aldehyde Dehydrogenases in Ethanol Metabolism in *Saccharomyces cerevisiae*", Wang et al, Molecular Cloning, 1998, Journal of Bacteriology, p. 822 - 830). Pro Mol umgesetztes Kosubstrat würden in diesem Fall 2 Mol Reduktionsäquivalente (NADH) entstehen (vgl. Reaktionsschema 2).

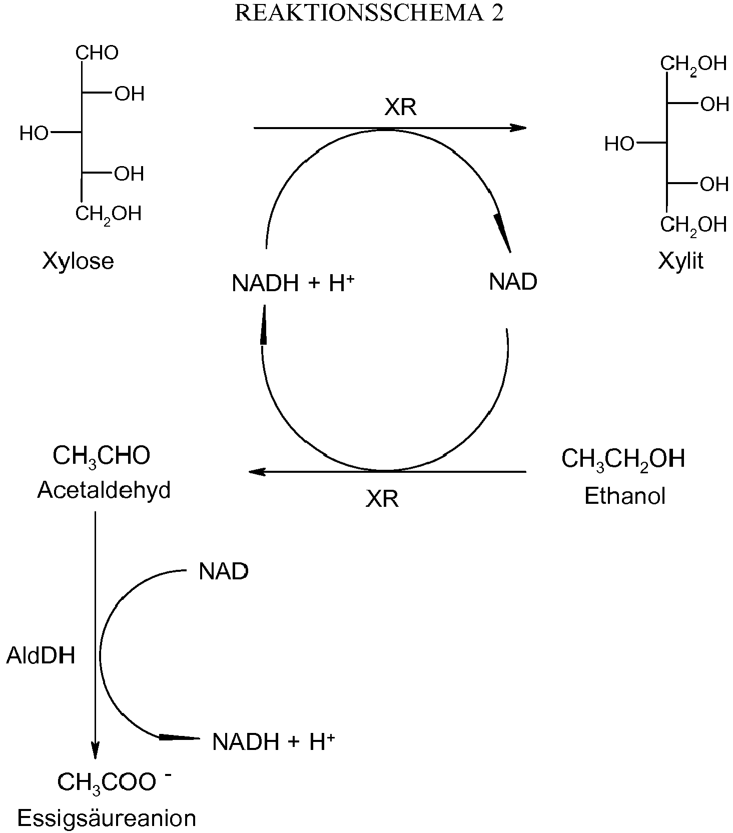

In der Tabelle 3 sind die Reaktionsverhältnisse der 4 veschiedenen Versuchsreaktionen 247, 249, 250 und 253 dargestellt. Es wurden unterschiedliche Ethanolkonzentrationen und AldDH-Konzentrationen verwendet. Die Kofaktor- und Subtratkonzentrationen wurden konstant gehalten.

**Tabelle 3**

| Reaktionsnummer | **247** | **249** | **250** | **253** |
|---|---|---|---|---|
| Substrat batch III [µL] | 300 (56 mM) | 300 (56 mM) | 300 (56 mM) | 300 (56 mM) |
| XR C. *tenius* 5U/mL [µL] | 25 (0,25 U/mL) | 25 (0,25 U/mL) | 25 (0,25 U/mL) | 25 (0,25 U/mL) |
| 20 mM NAD+[µL] | 10 (0,4 mM) | 10 (0,4 mM) | 10 (0,4 mM) | 10 (0,4 mM) |
| AldDH *S.cervisiae* 5U/mL [µL] | 25 (0,25U/mL) | 25 (0,25U/mL) | 0 | 0 |
| Ethanol 50% [µL] | 75 (1286 mM) | 70 (1200 mM) | 75 (1286 mM) | 70 (1200 mM) |
| 50 mM TrisHCl Puffer pH 7.0 [µL] | 65 | 70 | 90 | 95 |

Gesamtvolumen: 0,5 mL
Temperatur: 25 ± 2°C
Thermomixer: 500 rpm
Zeit: 112 Stunden

Zur Deaktivierung der Enzyme wurden alle Proben für 15 Minuten auf 70°C aufgeheizt und als Vorbereitung für die anschließende HPLC-Analyse zentrifugiert und filtriert (PVDF; 0,2 µm).

### Analyse - HPLC:

Säule SUGAR SP0810 + Vorsäule SUGAR SP-G
Säulentemperatur: 90°C
Detektor: Refraktionsindex-Detektor
Eluent: entionisiertes H₂O
Fluss: 0.90 mL/min
Probenmenge: 10 µL
HPLC Quantifizierung Präzision: ±10%

### Ergebnisse:

Die maximalen Ausbeute (Reaktion 249) konnte mit einer Ethanolkonzentration von 1,2 Mol/L erreicht werden. Dabei wurden insgesamt 1,38 mg/mL Xylitol erzeugt, was einer Ausbeute von 21,2% der Theorie an Xylitol entspricht.

In der Tabelle 4 sind die Ergebnisse der Reaktionen basierend auf den HPLC-Messdaten zusammengefasst.

**Tabelle 4**

| Reaktionsnummer | **247** | **249** | **250** | **253** |
|---|---|---|---|---|
| Theoretische Gesamtkonzentration [mg/mL] | 6,288 | 6,407 | 6,268 | 6,150 |
| Xylose nach der Reaktion [mg/mL] | 5,057 | 5,046 | 5,385 | 5,365 |
| Xylose verbraucht in der Reaktion [mg/mL] | 1,231 | 1,361 | 0,883 | 0,785 |
| | | | | |
| Gewinnung von Xylitol [mg/mL] | 1,248 | 1,379 | 0,894 | 0.796 |

Aus den Ergebnissen ist ersichtlich, dass Ethanol als Kosubstrat verwendet werden kann. Wie durch den Vergleich der Reaktionen 249 (Reaktionsgemisch beinhaltet AldDH) und 253 (Reaktionsgemisch ohne AldDH) eindeutig gezeigt werden kann, führt die Zugabe der Aldehyd-Dehydrogenase zu einer deutlichen Erhöhung der Ausbeute an Xylitol. Der Unterschied an umgesetzter Xylose zu Xylitol beträgt ∼8%. Dieses Ergebnis in Verbindung mit den oben erwähnten Literaturzitaten lässt nur den Schluss zu, dass AldDH den in der ersten Teilreduktion entstehenden Acetaldehyd weiter zu Essigsäure oxidiert (vgl. Reaktionsschema 2). Diese energetisch günstige Reaktion und die damit einhergehende erhöhte Konzentration an NADH verschiebt das Gleichgewicht vom Edukt in Richtung des Produktes Xylitol in der ersten Teilreaktion.

Die vorliegende Anmeldung offenbart weiters folgende Gegenstände:
1. Verfahren zur Herstellung von Kohlenhydratspaltprodukten, insbesondere Zuckern, gekennzeichnet durch die Kombination der Maßnahmen, dass
   - lignocellulosisches Material mit einer wässrigen Lösung, welche einen Alkohol, insbesondere einen C₁₋₄-Alkohol oder ein Phenol, enthält und einen pH-Wert zwischen 11,0 und 14,0 aufweist, behandelt wird, um Lignocellulose zu spalten und Spaltprodukte aus dem Material abzutrennen, wobei ein mit Cellulose und Hemicellulose angereichertes Material erhalten wird, und
   - das erhaltene, mit Cellulose und Hemicellulose angereicherte Material mit mindestens einem Kohlenhydrat-spaltenden Enzym behandelt wird, um die Kohlenhydratspaltprodukte zu gewinnen.
2. Verfahren nach Punkt 1, dadurch gekennzeichnet, dass die wässrige Lösung einen pH-Wert zwischen 11,0 und 13,0 aufweist.
3. Verfahren nach einem der Punkte 1 oder 2, dadurch gekennzeichnet, dass die Spaltung bei einer Temperatur von unter 100°C erfolgt.
4. Verfahren nach Punkt 3, dadurch gekennzeichnet, dass die Spaltung bei einer Temperatur von unter 40°C erfolgt.
5. Verfahren nach einem der Ansprüche 1 bis 4, dadurch gekennzeichnet, dass als lignocellulosisches Material Stroh, Bagasse, Energiegräser und/oder Spelzen eingesetzt wird.
6. Verfahren nach einem der Punkte 1 bis 5, dadurch gekennzeichnet, dass das lignocellulosische Material in der wässerigen Lösung in einer Stoffdichte von 5-40 Gew% vorliegt.
7. Verfahren nach einem der Punkte 1 bis 6, dadurch gekennzeichnet, dass das mit Cellulose und Hemicellulose angereicherte Material mit einer Xylanase und/oder einer Cellulase behandelt wird, um die Zucker zu gewinnen.
8. Verfahren nach einem der Punkte 1 bis 7, dadurch gekennzeichnet, dass die erhaltenen Zucker zu Alkohol vergoren werden, welcher abgetrennt und gewonnen wird.
9. Verfahren nach einem der Punkte 1 bis 8, dadurch gekennzeichnet, dass der aufgeschlossene Feststoff mit einer Xylanase umgesetzt und die erhaltene Flüssigphase zu Xylitol umgesetzt, und der verbleibende Feststoff
   - weiter mit Cellulase zum Erhalt verschiederner Fermentationsprodukte umgesetzt wird; oder
   - einer thermischen oder thermochemischen Stoffumwandlung unterzogen wird; oder
   - einer mikrobiellen Stoffumwandlung durch Bakterien, Hefen oder Pilze unterzogen wird;
      oder
   - einem weiteren Delignifizierungsschritt zum Zwecke der Gewinnung eines Cellulose-Fasermaterials unterzogen wird.
10. Verfahren nach Punkt 9, dadurch gekennzeichnet, dass der aufgeschlossene Feststoff mit einer Xylanase umgesetzt und die erhaltene Flüssigphase mittels Xylose Dehydrogenase zu Xylitol umgesetzt, und der verbleibende Feststoff
   - weiter mit Cellulase zum Erhalt verschiedener Fermentationsprodukte umgesetzt wird; oder
   - einer thermischen oder thermochemischen Stoffumwandlung unterzogen wird;
      oder
   - einer mikrobiellen Stoffumwandlung durch Bakterien, Hefen oder Pilze unterzogen wird;
      oder
   - einem weiteren Delignifizierungsschritt zum Zwecke der Gewinnung eines Cellulose-Fasermaterials unterzogen wird.
11. Verfahren nach einem der Punkte 9 oder 10, dadurch gekennzeichnet dass der nach Abtrennung der (Fermentations)Produkte verbleibende Feststoff in einer Biogasanlage fermentiert und zu Biogas weiterverarbeitet wird.

## Patentansprüche

1. Verfahren zur Herstellung von Xylitol, **gekennzeichnet durch** die Kombination der Maßnahmen, dass
- lignocellulosisches Material mit einer wässrigen Lösung, welche einen Alkohol, insbesondere einen C₁₋₄-Alkohol oder ein Phenol, enthält und einen pH-Wert zwischen 11,0 und 14,0 aufweist, behandelt wird, um Lignocellulose zu spalten, und Spaltprodukte aus dem Material abzutrennen, wobei ein mit Cellulose und Hemicellulose angereichertes Material erhalten wird,
- das erhaltene, mit Cellulose und Hemicellulose angereicherte Material mit Xylanase behandelt wird um Xylose zu gewinnen, und
- die Xylose enzymatisch zu Xylitol umgewandelt wird.

2. Verfahren nach Anspruch 1, **dadurch gekennzeichnet, dass** die wässrige Lösung einen pH-Wert zwischen 11,0 und 13,0 aufweist.

3. Verfahren nach einem der Ansprüche 1 oder 2, **dadurch gekennzeichnet, dass** die Spaltung bei einer Temperatur von unter 100°C erfolgt.

4. Verfahren nach einem der Ansprüche 1 bis 3, **dadurch gekennzeichnet, dass** als lignocellulosisches Material Stroh, Bagasse, Energiegräser und/oder Spelzen eingesetzt wird.

5. Verfahren nach einem der Ansprüche 1 bis 4, **dadurch gekennzeichnet, dass** das lignocellulosische Material in der wässerigen Lösung in einer Stoffdichte von 5-40 Gew% vorliegt.

6. Verfahren nach einem der vorhergehenden Ansprüche, **dadurch gekennzeichnet, dass** Glucose, Xylose und Lignin in getrennten Stoffströmen gewonnen werden.

7. Verfahren nach einem der vorhergehenden Ansprüche, **dadurch gekennzeichnet, dass** die enzymatische Umwandlung von Xylose zu Xylitol direkt im nach enzymatischer Freisetzung der Xylose aus dem mit Cellulose und Hemicellulose angereicherten Material erhaltenen Feststoff/Flüssigkeitsgemisch durchgeführt wird.

8. Verfahren gemäß einem der Ansprüche 1 bis 6, **dadurch gekennzeichnet, dass** nach der Behandlung mit der einen Alkohol enthaltenden wässerigen Lösung die Lignin enthaltende Lösung abgetrennt wird, der aufgeschlossene Feststoff mit einer Xylanase behandelt wird und die Flüssigphase vom Feststoff abgetrennt wird.

9. Verfahren nach einem der vorhergegangenen Ansprüche, **dadurch gekennzeichnet, dass** die Behandlung mit Xylanase im Temperaturbereich zwischen 30°C und 90°C durchgeführt wird.

10. Verfahren nach einem der vorhergehenden Ansprüche, **dadurch gekennzeichnet, dass** das mit Cellulose und Hemicellulose angereicherte Material mit einer Xylanase umgesetzt und die erhaltene Flüssigphase enzymatisch zu Xylitol umgesetzt wird, und der verbleibende Feststoff
- weiter mit Cellulase zum Erhalt verschiedener Fermentationsprodukte umgesetzt wird; oder
- einer thermischen oder thermochemischen Stoffumwandlung unterzogen wird;
oder
- einer mikrobiellen Stoffumwandlung durch Bakterien, Hefen oder Pilze unterzogen wird;
oder
- einem weiteren Delignifizierungsschritt zum Zwecke der Gewinnung eines Cellulose-Fasermaterials unterzogen wird.

11. Verfahren nach Anspruch 10, **dadurch gekennzeichnet, dass** das mit Cellulose und Hemicellulose angereicherte Material mit einer Xylanase umgesetzt und die erhaltene Flüssigphase mittels Xylosereduktase zu Xylitol umgesetzt, und der verbleibende Feststoff
- weiter mit Cellulase zum Erhalt verschiedener Fermentationsprodukte umgesetzt wird; oder
- einer thermischen oder thermochemischen Stoffumwandlung unterzogen wird;
oder
- einer mikrobiellen Stoffumwandlung durch Bakterien, Hefen oder Pilze unterzogen wird;
oder
- einem weiteren Delignifizierungsschritt zum Zwecke der Gewinnung eines Cellulose-Fasermaterials unterzogen wird.

12. Verfahren nach einem der Ansprüche 10 oder 11, **dadurch gekennzeichnet dass** der nach Abtrennung der (Fermentations)Produkte verbleibende Feststoff in einer Biogasanlage fermentiert und zu Biogas weiterverarbeitet wird.
